# EUROPEAN PATENT SPECIFICATION

(11) **EP 4 294 301 B1**
(45) Date of publication and mention of the grant of the patent: **09.04.2025**
(21) Application number: 21847898.0
(22) Date of filing: 29.12.2021
(51) Int. Cl.: A61B 18/12, A61B 18/14, A61B 18/16

(54) **CONTACT QUALITY SYSTEM**
KONTAKTQUALITÄTSSYSTEM
SYSTÈME DE QUALITÉ DE CONTACT

(30) Priority: 18.02.2021 US 202163150818 P
(43) Date of publication of application: 27.12.2023
(73) Proprietor: St. Jude Medical, Cardiology Division, Inc., St. Paul MN 55117 (US)
(72) Inventor: MASSMANN, Samuel P., Minneapolis, Minnesota 55408 (US)
(74) Representative: Mathys & Squire
(86) International application number: PCT/US2021/065478
(87) International publication number: WO 2022/177640

(56) References cited:
- EP-A1- 1 902 684
- US-A1- 2009 275 827
- US-A1- 2011 204 903
- US-A1- 2016 220 298
- US-A1- 2020 261 142
- US-A1- 2020 323 577

## Description

### BACKGROUND

The present disclosure relates to systems and methods of monitoring contact quality of return electrodes, such as body surface electrodes, during an ablation procedure.

Various therapies are used to treat various conditions afflicting the human anatomy. Cardiac arrhythmias, for example are sometimes treated using ablation therapy. When tissue is ablated, or at least subjected to ablative energy generated by an ablation generator and delivered by an ablation catheter, lesions form in the tissue. Electrodes mounted on or in ablation catheters are used to create tissue necrosis in cardiac tissue to correct conditions such as atrial arrhythmia (including, but not limited to, ectopic atrial tachycardia, atrial fibrillation, and atrial flutter). Arrhythmia (i.e., irregular heart rhythm) can create a variety of dangerous conditions including loss of synchronous atrioventricular contractions and stasis of blood flow that can lead to a variety of ailments and even death. It is believed that the primary cause of atrial arrhythmia is stray electrical signals within the left or right atrium of the heart. The ablation catheter imparts ablative energy (e.g., radiofrequency (RF) energy, cryoablation, lasers, chemicals, high-intensity focused ultrasound, etc.) to cardiac tissue to create a lesion in the cardiac tissue. This lesion disrupts undesirable electrical pathways and thereby limits or prevents stray electrical signals that lead to arrhythmias.

RF ablation systems include an RF source and a return. The RF source is generally a small electrode on a tip portion of a catheter, and the return can be one or more return electrodes (e.g., adhesive surface patches) disposed on the body and electrically coupled to a neutral or ground return node. The one or more return electrodes can be attached to the skin of the body using an adhesive or other fastening technique and have greater surface areas than the small electrode on a tip portion of a catheter. A decrease in contact quality can occur if the return electrode loses adhesion to the skin or body during a procedure.

US 2009/275827 A1 relates to a system for generating an excitation signal used in impedance measurements and for resolving the detected impedance. The magnitude of impedance reflects an entire impedance between an ablation tip electrode and a body patch electrode to which the excitation signal has been supplied.

US 2020/261142 A1 relates to a system for assessing a level of quality of contact between a distal end portion of a medical instrument such as an ablation catheter and a target region wherein no contact quality of a surface electrode is determined.

US 2016/220298 A1 relates toward an electrode catheter for tissue ablation, wherein no surface body electrode is excited and no signal is received in response to the excitation signal for determining a contact of the surface body electrode.

US 2011/204903 A1 relates to electrosurgical apparatuses comprising two separate electrode pads which are insulated from each other.

US 2020/323577 A1 relates to a system for monitoring electrode tissue engagement during ablation.

EP 1 902 684 A1 relates to a system for using a plurality of return electrodes during electrosurgery, in particular to the problem of reducing the risk of burning a patient in the area under the adhered portion of a return electrode.

### SUMMARY

The invention is defined by the appended claims, and relates to a system for monitoring contact quality of a first body surface electrode. The system includes a generator configured to provide ablation energy, an electronic control unit (ECU) configured to be in electrical communication with the first body surface electrode and the generator, and a circuit connecting the first body surface electrode to the ECU. The ECU is configured to send an interrogation signal through the circuit to the first body surface electrode and receive a sense signal through the circuit in response to the interrogation signal. The ECU determines the contact quality of the first body surface electrode by processing the sense signal.

According to the invention, the interrogation signal is provided at a first frequency if the ablation energy is being provided and at a second frequency if the ablation energy is not being provided. In some embodiments, the first body surface electrode comprises a first conductive portion and a second conductive portion, and the first conductive portion is separated from the second conductive portion by a gap. The interrogation signal can be provided across the gap, and the sense signal can have a voltage corresponding to an impedance across the gap.

Some embodiments relate to a system that includes a second body surface electrode and a variable impedance circuit coupled in series with at least one of the first body surface electrode and the second body surface electrode. The system also includes a first current sensor configured to sense a first current level associated with the ablation energy provided by the generator through the first body surface electrode, and a second current sensor configured to sense a second current level associated with the ablation energy provided by the generator through the second body surface electrode. The circuit is coupled to the first current sensor and the second current sensor. The circuit provides a control signal to the variable impedance circuit to balance the first current level through the first body surface electrode and the second current level through the second body surface electrode.

In some embodiments, the first frequency is lower than the second frequency, the first frequency being between 10 kilohertz (kHz) and 20 kHz and the second frequency being between 20 kHz and 200 kHz. In some embodiments, the circuit provides a quality signal in response to the sense signal being in a first relationship with a threshold and dynamically adjusts the threshold in response to a current level associated with the ablation energy provided by the generator through the first body surface electrode.

Some embodiments relate to a method of determining contact quality of a first body surface electrode attached to a body. The method includes providing an interrogation signal to the first body surface electrode. The interrogation signal is provided at a frequency. The frequency is determined in response to whether ablation energy is or is not being provided to the body. The method also includes receiving a sense signal from the first body surface electrode in response to the interrogation signal and processing the sense signal to determine the contact quality.

In some embodiments not part of the claimed subject-matter, a method can balance current through a first and second body surface electrode. The method includes sensing a first current level associated with the ablation energy through the first body surface electrode, sensing a second current level associated with the ablation energy provided through the second body surface electrode, and balancing current through the first body surface electrode and through the second body surface electrode in response to the first current level and the second current level.

### BRIEF DESCRIPTION OF THE DRAWINGS

The disclosure will become more fully understood from the following detailed description, taken in conjunction with the accompanying figures, wherein like reference numerals refer to like elements, in which:
FIG. 1 is a schematic drawing of a system for navigating and operating a medical device within a body according to some embodiments;
FIG. 2 is a general block diagram of an electronic control unit (ECU) for the system illustrated in FIG. 1 according to some embodiments;
FIG. 3 is a general block diagram of the contact quality monitor illustrated in FIG. 2 coupled to two return electrodes according to some embodiments;
FIG. 4 is a more detailed block diagram of the contact quality monitor illustrated in FIG. 3 according to some embodiments-; and
FIG. 5 is a flow diagram showing exemplary operations of the system illustrated in FIG. 1 according to some embodiments.

### DETAILED DESCRIPTION

The present disclosure relates to a dynamically configurable contact quality monitor system and method. The present disclosure also relates to a contact quality monitor or control system and method that actively balances current through two or more return electrodes. In some embodiments, the systems and methods are configured to adjust the frequency of interrogation signals provided to the return electrode to improve contact quality measurements and avoid interference. Higher frequency interrogation signals are used during non-treatment (e.g., non-ablation) periods to obtain a more reliable, consistent reading of contact quality in some embodiments. Lower frequency interrogation signals are used during treatment (e.g., ablation) periods to gain frequency separation, avoid interference and have continued monitoring during treatment periods in some embodiments. Advantageously, the contact quality systems and methods of some embodiments do not solely rely upon comparing a sense signal derived from interrogation signal to a fixed threshold to determine low contact quality (e.g., a 30-40% reduction in adhered patch area) of the return electrode according to some embodiments.

According to some embodiments, the contact quality systems and methods issue contact quality alarms or trips using the immediate RF current levels through the return electrode or electrodes. Advantageously, the contact quality systems and methods monitor individual current levels through two or more return electrodes and/or actively balance the current among the return electrodes. Details of the various examples of the present disclosure are described below with specific reference to the figures.

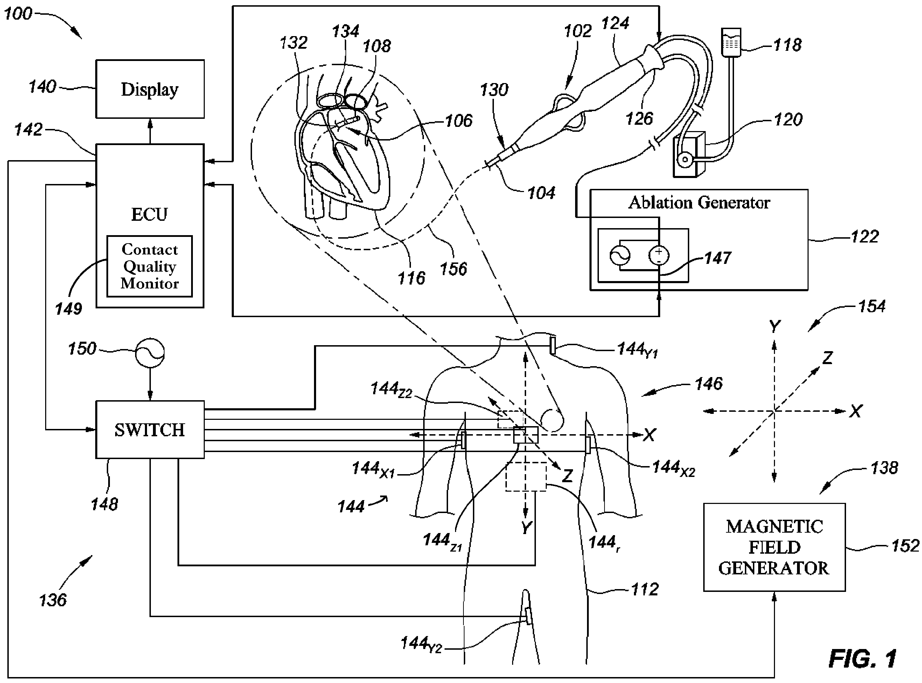

Referring to FIG. 1, one example of a system 100 for navigating and operating a medical device within a body 112 comprises a catheter 102, such as an ablation catheter, that is shown schematically entering a heart (e.g., tissue 116) that has been exploded away from the body 112. In some embodiments, catheter 102 can be from the TactiCath^{™} family of contact force ablation catheters, such as the TactiCath^{™} Contact Force Ablation Catheter, Sensor Enabled^{™} from Abbott Laboratories. It should be understood, however, that the system 100 can find application in connection with a wide variety of medical devices used within the body 112 for diagnosis or treatment. Further, it should be understood that the system 100 can be used to navigate medical devices used in the diagnosis or treatment of portions of the body 112 other than tissue 116 (e.g., cardiac tissue). Further description of the systems and components are contained in U.S. patent application serial no. 13/839,963 filed on 15 March 2013, now U.S. Patent No. 9,693 820.

The catheter 102 can include a handle 124, a cable connector or interface 126 at a proximal end of the handle 124, and a shaft 104 (also referred to herein as a catheter shaft). The shaft 104 can include a proximal end 130, and a distal end 132. A tip portion 106 can be located at the distal end 132. The handle 124 provides a location for the physician to hold the catheter 102 and can further provide means for steering or guiding the shaft 104 within the body 112. For example, the handle 124 can include means to change the length of one or more pull wires extending through the catheter 102 from the handle 124 to the distal end 132 of shaft 104. The construction of the handle 124 can vary.

The shaft 104 can be made from conventional materials such as polyurethane and can define one or more lumens configured to house and/or transport electrical conductors 156, fluids, or surgical tools. The shaft 104 can be introduced into a blood vessel or other structure within the body 112 through a conventional introducer. The shaft 104 can then be steered or guided through the body 112 to a desired location such as the tissue 116 using guide wires or pull wires or other means known in the art including remote control guidance systems. The shaft 104 can also permit transport, delivery, and/or removal of fluids (including irrigation fluids and bodily fluids), medicines, and/or surgical tools or instruments. It should be noted that any number of methods can be used to introduce the shaft 104 to areas within the body 112. This can include introducers, sheaths, guide sheaths, guide members, guide wires, or other similar devices. For ease of discussion, the term introducer will be used throughout.

In some examples, the system 100 can include a positioning system, a display 140, and an electronic control unit (ECU) 142. The ECU 142 can include, but is not limited to, a central processing unit (CPU), graphics processing unit (GPU), microprocessor, application specific integrated circuit (ASIC), a field programmable gate array (FPGA), complementary metal-oxide-semiconductor (CMOS), or the like. In some examples, the ECU can include memory, such as random-access memory (RAM), read-only memory (ROM), programmable read-only memory (PROM), erasable programmable read-only memory (EPROM), and electrically erasable programmable read-only memory (EEPROM), dynamic random-access memory (DRAM), static random-access memory (SRAM), Flash memory, or the like. The ECU 142 is a processor-based circuit in some embodiments.

The positioning system is a hybrid electric-field-based (impedance-based) and magnetic-field-based system including an electric-field-based positioning system 136 and a magnetic-field-based positioning system 138 in some embodiments. For example, the positioning system can be an EnSite Precision^{™} Cardiac Mapping System from Abbott Laboratories. The positioning system is provided to determine the position and orientation of the catheter 102, the tip portion 106, and similar devices within the body 112. For instance, the location or orientation of the tip portion 106 can be based on a fiducial or location of one or more electrodes of the tip portion 106, such as locational electrodes 134. In some examples, the locational electrodes 134 can include ring electrodes as shown in the example of FIG. 1. The system 136 operates based upon the principle that when low amplitude electrical signals are passed through the thorax, the body 112 acts as a voltage divider (or potentiometer or rheostat) such that the electrical potential or field strength measured at one or more electrodes, such as locational electrodes 134, on the catheter 102 can be used to determine the position of the catheter 102 relative to a pair of external patch electrodes using Ohm's law and the relative location of a reference electrode (e.g., in the coronary sinus).

In the configuration shown in FIG. 1, the electric-field-based positioning system 136 further includes three pairs of body surface electrodes 144. The three pairs of the body surface electrodes 144 are patch electrodes which are provided to generate electrical signals used in determining the position of the catheter 102 within a three-dimensional coordinate system 146 in some embodiments. The body surface electrodes 144 can also be used to generate electrophysiology (EP) data (e.g., electrophysiological signals) regarding the tissue 116. To create axes-specific electric fields within body 112, the body surface electrodes 144 are placed on opposed surfaces of the body 112 (e.g., chest and back, left and right sides of the thorax, and neck and leg) and form generally orthogonal X, Y, and Z axes. A reference electrode is typically placed near the stomach, provides a reference value and acts as the origin of the coordinate system 146 for the navigation system.

In accordance with this exemplary electric-field-based positioning system 136 as depicted in FIG. 1, the body surface electrodes 144 include right side patch 144_{X1}, left side patch 144_{X2}, neck patch 144_{Y1}, leg patch 144_{Y2}, chest patch 144_{Z1}, and back patch 144_{Z2}; and each body surface electrode 144 is connected to a switch 148 (e.g., a multiplex switch) and a signal generator 150. The body surface electrodes 144_{X1}, 144_{X2} are placed along a first (X) axis; the body surface electrodes 144_{Y1}, 144_{Y2} are placed along a second (Y) axis, and the body surface electrodes 144_{Z1}, 144_{Z2} are placed along a third (Z) axis. Sinusoidal currents are driven through each pair of body surface electrodes 144, and voltage measurements for one or more position sensors (e.g., locational electrodes 134) associated with the catheter 102 are obtained. The measured voltages are a function of the distance of the position sensors from the patch electrodes. The measured voltages are compared to the potential at the reference electrode and a position of the position sensors within the coordinate system 146 of the navigation system is determined.

In some embodiments, body surface electrodes 144 include at least one return electrode 144r for providing a return path to a return node 147 of the ablation generator 122 during ablation procedures. In some embodiments, the body surface electrodes 144 are provided in the Ensite^{™} NavX^{™} Surface Electrode Kit from Abbott Laboratories. In some embodiments, the return electrode 144r is one or more large surface area patches provided on a lower back of the body 112. In some embodiments, one or more of the body surface electrodes 144 discussed above can be configured for use as the return electrode 144r for ablation procedures. Exemplary configurations and uses of patch electrodes or body surface electrodes are discussed in U.S. Patent Application Publication No. 2017/0100055.

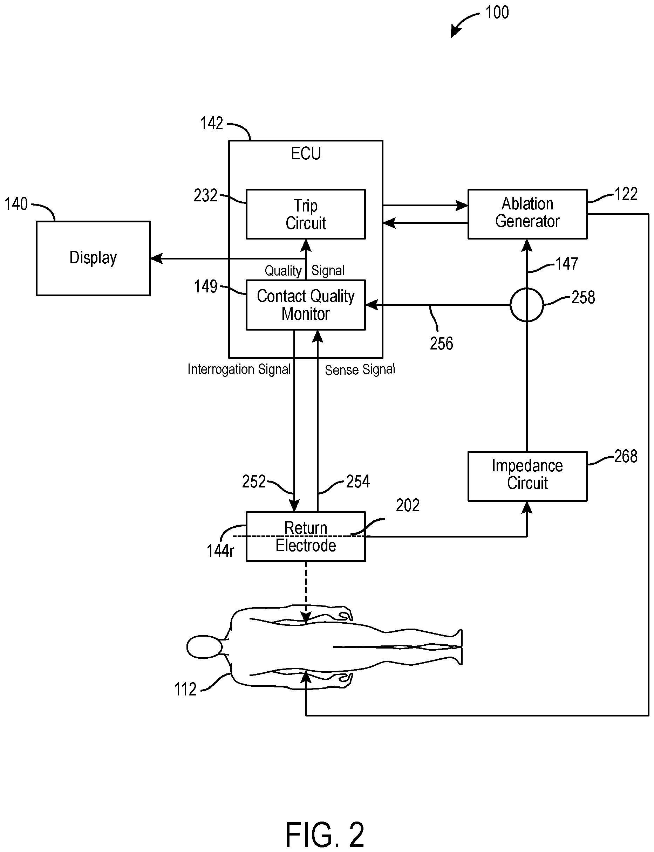

In some embodiments, the ECU 142 includes a contact quality monitor 149. In some embodiments, the ECU 142 is integrated into the ablation generator 122. Contact quality monitor 149 can be a standalone unit, part of switch 148, or other component of system 100 in some embodiments. The contact quality monitor 149 can be part of or integrated with one or more of the generator 122, the switch 148 and the ECU 142. Contact quality monitor 149 sends an interrogation signal across the return electrode 144r and processes a sense signal associated with the interrogation signal to determine impedance of the return electrode 144r through the skin/body and hence, the contact quality. The contact quality monitor 149 provides warnings and automatic tripping of the system 100 to shut off provision of ablation energy if the contact quality is insufficient in some embodiments. In some embodiments, the contact quality monitor 149 makes continuous measurements of current through each of multiple return electrodes 144r and adjusts the series impedance of each return path to actively balance the current through the return electrodes 144r. The operation of the contact quality monitor 149 is described in more detail below with respect to FIGS. 2-5 and can be used with any type of return electrodes.

The magnetic-field-based positioning system 138 in this example employs magnetic fields to detect the position and orientation of the catheter 102 within the body 112. In such a system, a magnetic field generator 152 can be employed having three orthogonally arranged coils (not shown) to create a magnetic field within the body 112 and to control the strength, orientation, and frequency of the field. The magnetic field generator 152 can be located above or below the patient (e.g., under a patient table) or in another appropriate location. Magnetic fields are generated by the coils and current or voltage measurements for one or more position sensors associated with the catheter 102 are obtained. The measured currents or voltages are proportional to the distance of the sensors from the coils, thereby allowing determination of a position of the sensors within a coordinate system 154 of system 138.

As the catheter 102 moves within the body 112, and within the electric field generated by the electric-field-based positioning system 136, the voltage readings from the locational electrodes 134 change, thereby indicating the location of catheter 102 within the electric field and within the coordinate system 146 established by the system 136. The locational electrodes 134 can be adapted to communicate position signals to the ECU 142.

The catheter 102 can be configured to deliver treatment as well as geometric modeling or electrophysiological mapping. In some examples, the catheter 102 can include at least one electrode 108 configured to detect electrophysiological signals from the tissue 116 or to provide energy for ablating the tissue 116. In an example, the at least one electrode 108 can be communicatively coupled to the ablation generator 122 for delivery of electrical signals adapted to provide the ablation energy to the at least one electrode 108. The ablation generator 122 can be an Ampere^{™} Generator from Abbott Laboratories and provides a sinusoidal electrical signal at 485 kilohertz (kHz) in some embodiments. Other types, frequencies, and forms of RF signals can be provided from the ablation generator 122. In some embodiments, at least one electrode 108 is an electrode assembly that may be used for a variety of diagnostic and therapeutic purposes including, for example and without limitation, cardiac mapping and/or ablation (e.g., RF ablation or Irreversible Electroporation (IRE ablation)/Pulsed Field Ablation (PFA)). The ablation generator 122 can be configured for bipolar PFA, which uses a train of bipolar and biphasic pulses of high voltage and short duration to create tissue modification without significant heating. For example, the electrode assembly may be configured as a bipolar electrode assembly for use in bipolar-based electroporation therapy. Specifically, a pair of electrodes are individually electrically coupled to an IRE generator (e.g., ablation generator 122 configured to be an IRE generator) and are configured to be selectively energized with opposite polarities to generate a potential and corresponding electric field there between for IRE therapy. That is, one of electrodes is configured to function as a cathode, and the other is configured to function as an anode. In some embodiments, the at least one electrode 108 may configured as a monopolar electrode assembly and use a patch electrode (as a return or indifferent electrode) (e.g., the return electrode 144r). The electrodes can provide ablation energy for IRE treatment by delivering electric current as a pulsed electric field (*i.e.,* PFA) in the form of ultrashort pulses (2-5 microsecond (µs) pulse width and 20-40 µs pulse period) grouped in bursts of 100-1000 pulses. In some embodiments, monopolar IRE ablation energy is provided at voltages of 2.5kV resulting in peak pulse currents up to 100A.

In some examples, the catheter 102 can be optionally connected to a fluid source 118 for delivering a biocompatible irrigation fluid such as saline through a pump 120. The pump 120 can include a fixed rate roller pump or variable volume syringe pump with a gravity feed supply from fluid source 118 as shown. The connector or interface 126 provides mechanical, fluid, and electrical connections for conduits or cables extending from the pump 120 and the ablation generator 122. The catheter 102 can also include other conventional components not illustrated herein such as a temperature sensor, additional electrodes, and corresponding conductors or leads. In some embodiments, the tip portion 106 includes tip electrodes associated with a grid catheter or basket catheter.

The ECU 142 provides a device for controlling the operation of various components of the system 100, including the catheter 102, the ablation generator 122, and magnetic field generator 152 of the magnetic-field-based positioning system 138. The ECU 142 can also provide a device for determining electrophysiology characteristics (e.g., signals) of the tissue 116, the position and orientation of the catheter 102 relative to tissue 116 and the body 112, controlling the ablation of the tissue 116, or any combination thereof. The ECU 142 also provides a device for generating display signals used to control the display 140.

The display 140 is provided to convey information to a physician to assist in diagnosis and treatment. The display 140 can comprise one or more conventional computer monitors or other display devices. The display 140 can present a graphical user interface (GUI) to the physician. The GUI can include a variety of information including, for example, contact quality information, warnings, or alarms, an image of the geometry of the tissue 116, electrophysiology data (e.g., maps of signals from the electrode 108) associated with the tissue 116, graphs illustrating voltage levels over time for various locational electrodes 134, and images of the catheter 102 and other medical devices and related information indicative of the position of the catheter 102 and other devices relative to the tissue 116.

Referring to FIG. 2, the ECU 142 includes the contact quality monitor 149 and a trip circuit 232. The ECU 142 is coupled to the display 140 and the ablation generator 122. The contact quality monitor 149 is coupled to one or more body surface electrodes 144 to provide a treatment to body 112 (e.g., human or animal). At least one return electrode144r is provided in a return path from the body 112 to the return node 147 of the ablation generator 122 in some embodiments.

The return electrode 144r can be a single return electrode embodied as a patch in some embodiments. The return electrode 144r can be two or more return electrodes embodied as two or more patches in some embodiments. In some embodiments, the return electrode 144r can be of various shapes, such as, square, rectangular, circular, trapezoidal, etc. In some embodiments, the return electrode 144r includes conductive portions electrically separated by a split or gap so that contact quality can be determined based upon impedance associated with the split as explained below. The return electrode 144r can be an adhesive, conductive structure that is applied to the skin on the lower back or other parts of the body 112. The return electrode 144r can be a surface body electrode as described in U.S. Patent Application Publication No. 2017/0100055 and provides a good electrical contact to the skin of the patient's body 112. Various conductive materials and layers can be used in the return electrode 144r, such as, silver carbon film, metal films, conductive adhesives or gels, a conductive foam sponge material, barrier layers, etc. Other methods of transferring electrical energy such as capacitive coupling of a conductive element to the patient's skin may also be practiced.

The contact quality monitor 149 is also coupled to a current transformer 258 and a variable impedance circuit 268. In some embodiments, the contact quality monitor 149 is a monitor or control circuit that performs at least one of the following operations: 1) providing an interrogation signal at a first frequency in a range between 20 kHz and 200 kHz in a first period when ablation is not being performed and at a second frequency in a range between10 kHz and 20 kHz in a second period when ablation is being performed; and 2) providing a signal indicating low contact quality in response to sense signal being in a relationship with a threshold, the threshold being set in response to a current level used during ablation.

In some embodiments, the contact quality monitor 149 is a circuit (e.g., processor - based circuit) including a conductor or lead 252 that provides an interrogation signal to the return electrode 144r. In some embodiments, the return electrode 144r is a single patch with a gap or a split 202 electrically dividing the conductive medium of the return electrode 144r. The interrogation signal is provided at a conductor, lead, or output 252 and travels across the split 202 via the contact between the skin and the return electrode 144r. A conductor, lead, or input 254 provides the sense signal from the return electrode 144r to the contact quality monitor 149. Output 252 and input 254 provide an interface for providing and receiving the interrogation signals and the sense signals and can include more than one conductor. The sense signal is derived from the interrogation signal and is representative of the impedance across the split 202 and hence the contact quality. In some embodiments, the interrogation signal is a constant current signal provided across the split 202, and the sense signal is a voltage measurement across the split 202 representative of impedance across the split 202. The sense signal is compared to a threshold to determine if the contact quality is sufficient for the procedure. In some embodiments, the contact quality monitor 149 provides a quality signal to the trip circuit 232 as well as other parts of the ECU 142. In some examples, the ECU 142 can provide alarms, warnings, or other contact information for display on the display 140 in response to the quality signal.

The trip circuit 232 shuts down, disables, or prevents the provision of the ablation energy by the ablation generator 122 when the quality signal indicates that the contact quality is not sufficient (e.g., a 30-40 percent reduction in adhered patch area). The trip circuit 232 includes a switch that isolates the ablation generator 122 from the body 112 in some embodiments. In some embodiments, the trip circuit 232 is part of the contact quality monitor 149 and provides an interrupt or flag to the ECU 142 in the event of insufficient contact quality.

In some embodiments, the interrogation signal is provided in a lower frequency range (e.g., 10-20 kHz) during ablation to avoid interference with the higher frequency signals from the ablation generator 122. This separation in frequency also allows, with standard electronic components, a low-impedance path for the return of energy to the return node 147 and the higher impedance path for the interrogation signal in some embodiments. Additionally, capacitive components of the impedance of the body 112 are more pronounced at lower frequencies, which can result in additional inconsistencies in the measurements of contact quality. Accordingly, the interrogation signal is provided at a higher frequency in a higher frequency range (e.g., 20-200 kHz) when ablation is not provided to provide a more consistent, accurate measurement of impedance and contact quality.

The current concentration I_{con} is found by taking the total patch current I_{patch} divided by the adhered patch area A_{efrective} (assuming there is uniform distribution of current through the effective patch area) (I_{con}= I_{patch}/A_{effective}). Therefore, if the patch current is low, there is a lower minimum adhered area required for proper current concentration.

In some embodiments, the quality signal indicates insufficient contact quality when impedance is higher than a threshold representing impedance associated with insufficient conatc quality. The threshold is not a fixed threshold and is adjusted according to the current or power level received at the return node 147 in some embodiments. The threshold is decreased when the current level is higher because a higher level of contact quality (lower impedance) may be needed during higher current ablation procedures in some embodiments. A lower level of contact quality is tolerable during lower current procedures in some embodiments. In some embodiments, the contact quality monitor 149 uses an algorithm to continuously adjust the threshold for contact quality (adhered patch area) based on the amount of current through the return electrode 144r.

The current transformer 258 provides a power sense signal or current level signal to the contact quality monitor 149 via a lead 256. The current transformer 258 can be any device for sensing power or current in the conductor associated with the return node 147 in some embodiments. The peak current level used during the procedure can be used to adjust the threshold in some embodiments. In some embodiments, the peak current level in a period of time is used. In some embodiments, real time current level is used to adjust the threshold. In some embodiments, the current level is provided by ECU 142 based upon system configuration or user input. In some embodiments, the threshold is also adjusted based upon whether the higher frequency interrogation signal is provided when ablation is not being performed or whether the lower frequency interrogation signal is provided when ablation is performed (e.g., a threshold specific for each of the higher and lower frequency interrogation signals). In some embodiments, a magnitude of the threshold is inversely related to the current level associated with the ablation energy provided by the ablation generator 122 through the return electrode 144r.

In some embodiments, the threshold represents a threshold impedance for low contact quality. The sense signal is a voltage signal that represents impedance (e.g., the higher the sense signal the higher the impedance) in some embodiments. When the sense signal is above the threshold, a low contact quality signal is provided. The low contact quality signal can be used to provide warnings or trip the system 10. Conversely, a signal representing sufficient contact quality can be provided when the sense signal is below the threshold. The threshold can be selected or adjusted for a particular type of return electrode 144r, the number of return electrodes 144r, the location of the return electrode 144r on the body 112, or the type of procedure. The particular type of return electrode 144r, the number of return electrodes 144r, and the location of the return electrode 144r can be sensed or input as data by a user.

In some embodiments, the threshold is fixed and represents current concentration. The actual current concentration I_{con} is compared to the threshold where actual current concentration I_{con} is calculated using the sense signal (representing A_{effective}) and the current level from the current transformer 258 (Ip_{atch})( I_{con}= I_{patch}/A_{effective}). Using current concentration I_{con} or a dynamic threshold is advantageous, because the number of situations in which the output is inappropriately disabled is reduced.

The contact quality signal is also provided to the ECU 142 for audible or visual warning of a low contact quality. The warning can be provided in accordance with the threshold in some embodiments. A warning or caution can also be provided if a change in impedance becomes higher even though the threshold is not reached. A quickly increasing impedance can indicate that adjustment of the return electrode 144r may be required in some embodiments. The display 140 can also provide an analog indication of contact quality based upon the contact quality signal or the sense signal.

In some embodiments, the contact quality monitor 149 adjusts the impedance of the impedance circuit 268 between the return electrode 144r and return node 147. The impedance circuit 268 includes one or more of active devices, resistors, capacitors or inductors for adjusting impedance via switched paths, active control or other control technique.

The ECU 142 and contact quality monitor 149 are implemented as a circuit including one or more of a general purpose processor, a specific purpose processor, memory, application specific integrated circuits (ASICs), field programmable gate arrays (FPGAs), groups of processing components, interface, isolation, and filter components, analog and digital support circuits or other suitable electronic processing components. The memory is one or more storage devices (e.g., RAM, ROM, flash memory, hard disk storage) for storing data and computer code for completing and facilitating the various -processes, layers, and modules described in the present disclosure. The memory may be or include volatile memory or non-volatile memory and may include database components, object code components, script components, or any other type of information structure for supporting the various activities and information structures of the inventive concepts disclosed herein. The memory is communicably connected to the processor and includes computer code or instruction modules for executing one or more processes described herein. The memory includes various circuits, software engines, and/or modules that cause the one or more processors to execute the systems and methods described herein.

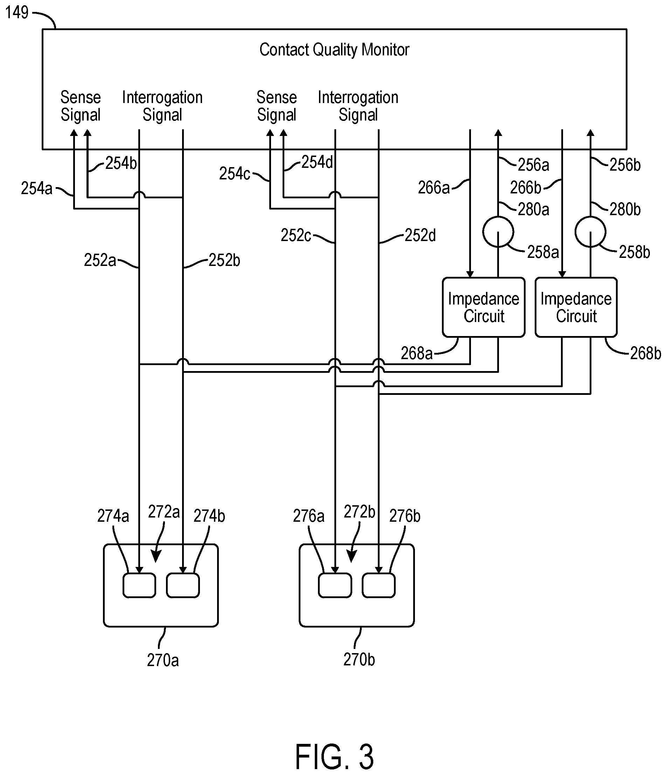

With reference to FIG. 3, the contact quality monitor 149 is coupled to the return electrode 144r (FIG. 2) embodied as two or more return electrodes 270a and 270b. Any number of return electrodes 144r can be used with the contact quality monitor 149 where each return electrode 144r is associated with a channel for a respective interrogation signals, sense signals, sensed current levels, impedance adjustment, etc. Return electrodes 270a and 270b are placed asymmetrically on the body. The placement can result in a lower impedance path through the body from the tip portion 106 (FIG. 1) to one of the return electrodes 270a and 270b over another of the return electrodes 270a and 270b. Due to the lower impedance path, one of the return electrodes 270a and 270b can receive more return energy than the other of the return electrodes 270a and 270b. This can create an imbalance in which one return electrode of the return electrodes 270a and 270b passes more current than the other return electrode of the return electrodes 270a and 270b. This can be undesirous in a high current mode where even distribution of return current is desired.

Return electrode 270a includes two conductive portions, 274a and 274b, separated by a gap or split 272a, and return electrode 270b includes two conductive portions, 276a and 276b, separated by a gap or a split 272b. A lead 252a is coupled to portion 274a, and a lead 252b is coupled to portion 274b. A lead 252c is coupled to portion 276a, and a lead 252d is coupled to portion 276b. A sense input 254a is coupled to portion 274a, and a sense input 254b is coupled to portion 274b. A sense input 254c is coupled to portion 276a, and a sense input 254d is coupled to portion 276b. Leads 252a-b are the signal and return nodes for the interrogation signal for return electrode 270a, and leads 252c-d are the signal and return nodes for the interrogation signal for return electrode 270b. Sense inputs 254a-b are for the sense signal for return electrode 270a, and sense inputs 254c-d are for the sense signal for return electrode 270b.

The sense signal across the sense inputs 254a and 254b is compared to a threshold to determine contact quality of the return electrode 270a. The threshold is dynamically adjusted based upon a current level sensed by the current transformer 258a. The sense signal across the sense inputs 254c and 254d is compared to a threshold to determine contact quality of the return electrode 270b. The sense signals at inputs 254a-b and 254c-d are voltage measurements of the interrogation signals across splits 272a-b in some embodiments. The threshold is dynamically adjusted based upon a current level sensed by the current transformer 258b. A contact quality signal can be provided for each of return electrodes 270a and 270b as well as a composite contact quality signal for both return electrodes 270a and 270b.

In some embodiment, the return paths for return electrodes 270a and 270b include respective impedance circuits 268a and 268b coupled to respective portions 274a-b and 276a-b. The impedance circuits 268a and 268b can be adjusted to balance return current between the return electrodes 270a and 270b. Leads 266a-b provide an interface to the contact quality monitor 149 and can be used to control impedance in the impedance circuits 268a-b in response to current level signals from current transformers 258a and 258b indicating current in the return electrodes 270a and 270b. If return electrode 270a is receiving more return current than return electrode 270b, the impedance of impedance circuit 268a in the return path of return electrode 270a can be increased or the impedance of impedance circuit 268b in the return path of electrode 270b can be decreased to balance the return current between return electrodes 270a and 270b.

Current transformers 258a and 258b provide current level signals via leads 256a-b indicative of the current in each return path. Leads 256a and 256b provide an interface to the contact quality monitor 149. A comparison of the current levels sensed by current transformers 258a-b is used to determine the amount of impedance adjustment. Advantageously, each return electrode 270a and 270b is connected to return node 147 (FIG. 1) through impedance circuits 268a-b. Impedance circuits 268a-b are coupled to portions 274a-b and 276a-b, respectively, and a respective single conductor 280a-b is provided through each of current transformers 258a-b to return node 147 (FIG. 1). Impedance circuits 268a-b are printed circuit boards (PCBs) with an adjustable LC (inductor-capacitor) resonant trap circuit and variable resistance R in some embodiments. In some embodiments, for components not adjustable through digital control, an array of relays is used to switch components that are connected in the return path to adjust the impedance. In some embodiments, return electrodes 270a and 270b can be any number of electrodes including three, four or more electrodes, each associated with a path or channel having a respective impedance circuit, current sensor, and interface for a sense signal and an interrogation signal.

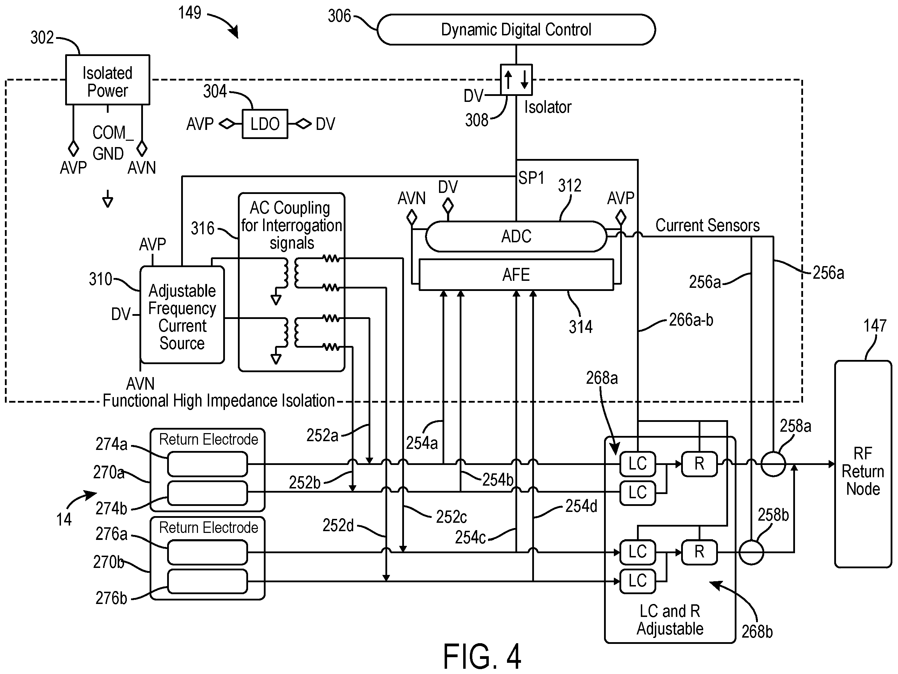

With reference to FIG. 4, the contact quality monitor 149 includes an isolated power circuit 302, a power converter 304, dynamic digital control circuit 306, an isolator 308, an adjustable frequency current source 310, an analog to digital converter 312, an analog front end circuit 314, and an isolation circuit 316 in some embodiments. In some embodiments, the contact quality monitor 149 is coupled to return electrodes 270a and 270b. Dynamic digital control circuit 306 is a processor or other circuit configured to provide the operations described herein with regard to contact quality monitoring and control. The analog to digital converter 312 provides digital data to the dynamic digital control circuit 306 representing sense signals from inputs 254a-d and current level signals from current transformers 258a-b.

Impedance circuits 268a and 268b are coupled in the return path of return electrodes 270a and 270b, respectively. Advantageously, each return electrode 270a and 270b is connected to return node147 through a respective impedance circuit 268a-b. Dynamic digital control circuit 306 controls impedance circuits 268a-b via digital control signals on leads 266a-b in some embodiments. Dynamic digital control circuit 306 provides the signals on the leads 266a-b for load balancing in response to the current level sense signals provided via leads 256a-b and analog to digital converter 312. Analog front end circuit 314 provides frequency conversion, filtering, conditioning and other interface functions before the sense signals are converted into digital data.

Dynamic digital control circuit 306 provides the interrogation signals at different frequencies depending upon whether ablation energy is provided (e.g., whether return node 147 is receiving current associated with signals from the ablation generator 122 (FIG. 1) in some embodiments). In some embodiments, dynamic digital control circuit 306 receives a signal or data from ECU 142 (FIG. 1) indicating that ablation energy is provided so that an appropriate frequency can be selected for the interrogation signal. The frequency may be chosen based upon the frequency of the electric signals for ablation to avoid interference, harmonic frequencies, etc. The ability to select a frequency for the interrogation signal that does not interfere with other equipment or components within system 100 is advantageous. For example, positioning systems or mapping systems can provide hundreds of impedance-measuring signals with frequencies between 8 and 9 kHz and 16 and 22 kHz, and the interrogation signal can be provided outside of these bands to avoid interference.

In some embodiments, the interrogation signals are provided via an alternating current coupling circuit such as isolation circuit 316. The adjustable frequency current source 310 is controlled digitally and drives an interrogation signal across the return electrodes 270a and 270b or a single patch electrode in some embodiments. The adjustable frequency current source 310 is a constant current source, and the interrogation signals are provided at a current between at 10-100 microamperes (uA) root mean square (RMS) in some embodiments. The resulting voltage across the return electrodes 270a and 270b is provided via inputs 254a-d through the analog front end circuit 314 as the sense signals to the analog to digital converter 312 for receipt by the dynamic digital control circuit 306. The analog front end circuit 314 is tuned to achieve proper performance at all usable frequencies (approximately 10kHz-100kHz) in some embodiments.

Power converter 304 is a drop out regulator in some embodiments. Dynamic digital control is a processor-based circuit, field programmable gate array, application specific circuit (ASIC), microcontroller, or combination thereof in some embodiments. An isolation barrier provides high impedance isolation from return electrodes 270a and 270b and return node 147. Isolation circuit 316 is a transformer-based circuit in some embodiments.

Although the example of FIG. 4 is shown with two return electrodes 270a and 270b, the contact quality monitor 149 can be configured for more than two return electrodes 270a and 270b by adding a channel for each additional return electrode. In some embodiments, the contact quality monitor 149 can be configured for a single return electrode 270a by removing the channel associated with the return electrode 270b.

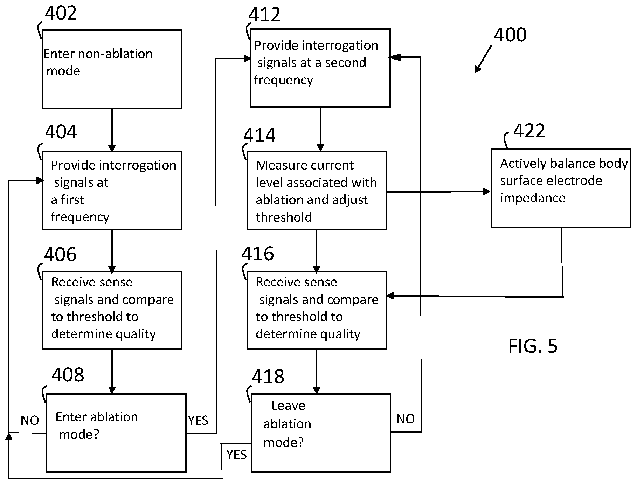

With reference to FIGS. 1 and 5, the system 100 and contact quality monitor 149 operate according to flow 400 in some embodiments. At an operation 402, system 100 is in a non-ablation mode where ablation energy is not being provided to the body 112 in some embodiments. The non-ablation mode can include a quiet mode in which the ablation generator 122 is powered on or not completely off and the system 100 is not providing ablation energy. In some embodiments, the ablation generator 122 can provide a low amplitude signal (e.g., at 485 kHz) for measurement purposes in the non-ablation mode.

At an operation 404, the contact quality monitor 149 provides interrogation signals at a first frequency. The first frequency is in a frequency range between 20 and 200 kHz in some embodiments. A higher frequency interrogation signal provides more accurate impedance readings and can be used during a non-ablation mode because the higher frequency, higher amplitude ablation signals are not present and therefore, do not interfere with the higher frequency interrogation signal. In some embodiments, the first frequency has an upper limit of approximately 100 kHz to avoid interference with signals from the ablation generator 122. In some embodiments, the first frequency is above 100 kHz or above 200 kHz signal and is chosen to avoid interference with signals from the ablation generator 122 (e.g., measurement signals such as the 485 kHz measurement signal). In some embodiments, the first frequency is provided at a frequency between 20-200 kHz that is selected to obtain accurate and stable patch impedance readings. The first frequency can be selected in accordance with system parameters and design criteria such as avoidance of signal interference from other signals in the system 100 and electrical characteristics (e.g., capacitance, inductance, and resistance of the return electrode 144r (FIG. 1) and filters), etc.

At an operation 406, the contact quality monitor 149 receives sense signals and compares the sense signals to a threshold to determine contact quality. The contact quality is used to provide alarms or trip operation of the system 100. The threshold can represent impedance and be adjusted lower in accordance with the amount of ablation energy provided during ablation treatment (e.g., a lower threshold for higher amounts of ablation energy).

At an operation 408, the contact quality monitor 149 returns to operation 404 if the ablation mode is not entered. The ablation mode is a period where ablation energy is provided to the body 112 in some embodiments. Entry into the ablation mode can be determined from a signal from ECU 142, a user input, or the presence of current associated with ablation energy at return electrode 144r.

If the ablation mode is entered, the contact quality monitor 149 advances to an operation 412. At operation 412, the contact quality monitor 149 provides provide interrogation signals at a second frequency lower than the first frequency. In some embodiments, the second frequency is between 5 and 50 kHz (e.g., 10-20 kHz). In some embodiments, the second frequency is 12 kHz. As the interrogation signal frequency gets closer to the frequency (e.g., 485 kHz ) of the ablation signal provided by the ablation generator 122, more noise or interference is present. In some embodiments, the second frequency is at least one logarithmic decade (order of magnitude) away from the frequency of the ablation signal (e.g., an approximately 50 kHz upper limit when the frequency of the ablation signal is approximately 500 kHz) so that RF noise can be effectively filtered out. The second frequency is high enough so that the impedance measurements are not inconsistent due to body/patch/skin capacitive effects showing up in the measurement. A lower frequency limit for the second frequency can be dependent on characteristics of the return electrode 144r (FIG. 1) and the body 112.

At an operation 412, the contact quality monitor 149 measures the current level associated with the ablation energy and adjusts the threshold in accordance with the current level. At an operation 422, the contact quality monitor 149 performs active balancing of body surface electrodes impedance in response to current levels through the return electrodes 144r (e.g., measured at operation 414). Operation 422 is used if two or more return electrodes 144r are used in system 100. Operation 422 is optional in some embodiments.

After operation 414 or 422, the contact quality monitor 149 receives sense signals and compares the sense signals to a threshold to determine contact quality in an operation 416. The threshold can be dynamically adjusted as discussed above. After operation 416 and at an operation 418, the contact quality monitor 149 returns to operation 404 if system 100 has not left the ablation mode. If the non-ablation mode is entered, the contact quality monitor 149 advances to operation 404. Operations 414 and 412 can be performed simultaneously with other operations in flow 400. After operations 406 and 416, ablation operations can be stopped if contact quality is insufficient.

Although embodiments are described above with respect to RF ablation treatment, RF ablation treatment is not disclosed in a limiting fashion. For example, the contact quality monitor 149 can be used with an IRE ablation treatment without departing from the scope of the claims. For example, a monopolar IRE system with two or more return electrodes 144r could use the current balancing technique of the contact quality monitor 149. In another example, a monopolar IRE system used with one or more return electrodes 144r could use the dynamic threshold technique of the contact quality monitor 149. Further, the contact quality monitor 149 can be used with other treatments and procedures that use one or more return electrodes 144r without departing from the scope of the claims. Although embodiments are described above with respect to cardiac treatment, contact quality monitor 149 can be used with systems designed to treat other organs. Although embodiments are described above with respect to catheter ablation treatment, contact quality monitor 149 can be used with non-catheter ablation systems.

It is to be understood that embodiments of the methods according to the concepts disclosed herein may include one or more of the steps described herein. Further, such steps may be carried out in any desired order and two or more of the steps may be carried out simultaneously with one another. Two or more of the steps disclosed herein may be combined in a single step, and in some embodiments, one or more of the steps may be carried out as two or more sub-steps. Further, other steps or sub-steps may be carried out in addition to, or as substitutes to one or more of the steps disclosed herein. Although processors and non-transitory computer media is disclosed, the circuits for performing the described operations can take other forms.

From the above description, it is clear that the inventive concepts disclosed herein are well adapted to carry out the objects and to attain the advantages mentioned herein as well as those inherent in the inventive concepts disclosed herein. While presently preferred embodiments of the inventive concepts disclosed herein have been described for purposes of this disclosure, it will be understood that numerous changes may be made which will readily suggest themselves to those skilled in the art and which are accomplished within the broad scope and coverage of the inventive concepts disclosed and claimed herein.

## Claims

1. A system for monitoring contact quality of a first body surface electrode, the system comprising:
a first body surface electrode (144r);
a generator (122) configured to provide ablation energy;
an electronic control unit (ECU) (142) configured to be in electrical communication with the first body surface electrode (144r) and the generator (122);
a circuit (149) connecting the first body surface electrode (144r) to the ECU (142);
wherein the ECU (142) is configured to send an interrogation signal through the circuit (149) to the first body surface electrode (144r) and receive a sense signal from the first body surface electrode (144r) through the circuit (149) in response to the interrogation signal; and
wherein the ECU (142) determines the contact quality of the first body surface electrode (144r) by processing the sense signal, **characterized in that**, the interrogation signal is provided at a first frequency if the ablation energy is being provided and at a second frequency if the ablation energy is not being provided.

2. The system of claim 1, wherein the first frequency is lower than the second frequency, or wherein the first frequency is between 10 kHz and 20 kHz and the second frequency is between 20 kHz and 100 kHz.

3. The system of claim 1, wherein the first body surface electrode (144r) comprises a first conductive portion and a second conductive portion, wherein the first conductive portion is separated from the second conductive portion by a gap, wherein the interrogation signal is sent across the gap and the sense signal has a voltage corresponding to an impedance across the gap.

4. The system of claim 3, wherein the circuit (149) provides a low contact quality signal when the sense signal is higher than a threshold, wherein the system includes a trip circuit (232) configured to shut down, disable, or prevent the provision of the ablation energy by the generator in response to the low contact quality signal.

5. The system of claim 1, further comprising a second body surface electrode, wherein the first body surface electrode and the second body surface electrode each comprise a first conductive portion and a second conductive portion, each first conductive portion being separated from each second conductive portion by a respective gap, wherein the circuit (149) provides respective interrogation signals to and receives respective sense signals from each first conductive portion and each second conductive portion.

6. The system of claim 5, wherein the circuit (149) provides each of the interrogation signals at a first frequency if the ablation energy is being provided by the generator and at a second frequency if the ablation energy is not being provided, the first frequency being below the second frequency, wherein the circuit (149) provides a low contact quality signal in response to at least one of the respective sense signals being higher than a threshold.

7. The system of claim 6, wherein the low contact quality signal indicates more than a 40 percent reduction in an adhered area from an expected adhered area of the first body surface electrode (144r) or the second body surface electrode.

8. The system of claim 1, further comprising:
a second body surface electrode;
a variable impedance circuit coupled in series with at least one of the first body surface electrode (144r) and the second body surface electrode;
a first current sensor configured to sense a first current level associated with the ablation energy provided by the generator through the first body surface electrode (144r);
a second current sensor configured to sense a second current level associated with the ablation energy provided by the generator through the second body surface electrode; and
wherein the circuit is coupled to the first current sensor and the second current sensor, wherein the circuit (149) provides a control signal to the variable impedance circuit to balance the first current level through the first body surface electrode and the second current level through the second body surface electrode.

9. The system of claim 1, wherein the circuit (149) comprises an adjustable frequency current source (310) for providing the interrogation signal.

10. The system of claim 1, wherein the circuit (149) provides a low contact quality signal when the sense signal is higher than a threshold.

11. The system of claim 10, wherein the circuit is configured to dynamically adjust the threshold in response to a current level associated with the ablation energy provided by the generator through the first body surface electrode (144r).

12. The system of claim 11, wherein the threshold is adjusted lower when the current level associated with the ablation energy provided by the generator (122) through the first body surface electrode (144r) is increased, or
wherein the circuit (149) comprises a current transformer disposed between a return node of the generator (122) and the first body surface electrode (144r), wherein the current level is determined using a current signal from the current transformer, or
wherein the circuit comprises an analog-to-digital converter for receiving the sense signal.

13. The system of claim 1, wherein the circuit comprises an adjustable impedance circuit in series with the first body surface electrode and a return node of the generator, wherein the adjustable impedance circuit is tuned to block signals in a first frequency range while allowing signals in a second frequency range to pass.

14. The system of claim 1, further comprising:
a catheter configured to provide the ablation energy to a body; wherein the first body surface electrode (144r) is a return electrode.

## Patentansprüche

1. Ein System zur Überwachung der Kontaktqualität einer ersten Körperoberflächenelektrode, wobei das System Folgendes umfasst:
eine erste Körperoberflächenelektrode (144r);
einen Generator (122), der dazu ausgelegt ist, Ablationsenergie bereitzustellen;
eine elektronische Steuereinheit (ESE) (142), die dazu ausgelegt ist, in elektrischer Verbindung mit der ersten Körperoberflächenelektrode (144r) und dem Generator (122) zu sein;
einen Stromkreis (149), der die erste Körperoberflächenelektrode (144r) mit der ESE (142) verbindet;
wobei die ESE (142) dazu ausgelegt ist, ein Abfragesignal durch den Stromkreis (149) an die erste Körperoberflächenelektrode (144r) zu senden und ein Erfassungssignal von der ersten Körperoberflächenelektrode (144r) durch den Stromkreis (149) als Reaktion auf das Abfragesignal zu empfangen; und
wobei die ESE (142) die Kontaktqualität der ersten Körperoberflächenelektrode (144r) durch Verarbeiten des Erfassungssignals bestimmt, **dadurch gekennzeichnet, dass** das Abfragesignal bei einer ersten Frequenz bereitgestellt wird, wenn die Ablationsenergie gerade bereitgestellt wird, und bei einer zweiten Frequenz bereitgestellt wird, wenn die Ablationsenergie gerade nicht bereitgestellt wird.

2. System nach Anspruch 1, wobei die erste Frequenz niedriger ist als die zweite Frequenz oder wobei die erste Frequenz zwischen 10 kHz und 20 kHz beträgt und die zweite Frequenz zwischen 20 kHz und 100 kHz beträgt.

3. System nach Anspruch 1, wobei die erste Körperoberflächenelektrode (144r) einen ersten leitenden Abschnitt und einen zweiten leitenden Abschnitt umfasst, wobei der erste leitende Abschnitt durch einen Spalt von dem zweiten leitenden Abschnitt getrennt ist, wobei das Abfragesignal über den Spalt gesendet wird und das Erfassungssignal eine Spannung aufweist, die einer Impedanz über den Spalt hinweg entspricht.

4. System nach Anspruch 3, wobei der Stromkreis (149) ein Signal mit geringer Kontaktqualität bereitstellt, wenn das Erfassungssignal höher als ein Schwellenwert ist, wobei das System einen Auslösestromkreis (232) einschließt, der dazu ausgelegt ist, die Bereitstellung der Ablationsenergie von dem Generator als Reaktion auf das Signal mit geringer Kontaktqualität abzuschalten, zu deaktivieren oder zu verhindern.

5. System nach Anspruch 1, das ferner eine zweite Körperoberflächenelektrode umfasst, wobei die erste Körperoberflächenelektrode und die zweite Körperoberflächenelektrode jeweils einen ersten leitenden Abschnitt und einen zweiten leitenden Abschnitt umfassen, wobei jeder erste leitende Abschnitt durch einen jeweiligen Spalt von jedem zweiten leitenden Abschnitt getrennt ist, wobei der Stromkreis (149) jeweilige Abfragesignale zu jedem ersten leitenden Abschnitt und jedem zweiten leitenden Abschnitt bereitstellt und jeweilige Erfassungssignale von jedem ersten leitenden Abschnitt und jedem zweiten leitenden Abschnitt empfängt.

6. System nach Anspruch 5, wobei der Stromkreis (149) jedes von den Abfragesignalen bei einer ersten Frequenz bereitstellt, wenn die Ablationsenergie gerade von dem Generator bereitgestellt wird, und bei einer zweiten Frequenz bereitstellt, wenn die Ablationsenergie gerade nicht bereitgestellt wird, wobei die erste Frequenz unter der zweiten Frequenz liegt, wobei der Stromkreis (149) ein Signal mit geringer Kontaktqualität als Reaktion darauf bereitstellt, dass mindestens eines der jeweiligen Erfassungssignale höher als ein Schwellenwert ist.

7. System nach Anspruch 6, wobei das Signal mit geringer Kontaktqualität eine mehr als 40-prozentige Reduktion in einer Anhaftfläche von einer erwarteten Anhaftfläche der ersten Körperoberflächenelektrode (144r) oder der zweiten Körperoberflächenelektrode anzeigt.

8. System nach Anspruch 1, das ferner Folgendes umfasst:
eine zweite Körperoberflächenelektrode;
einen variablen Impedanzstromkreis, der in Reihe mit mindestens einer von der ersten Körperoberflächenelektrode (144r) und der zweiten Körperoberflächenelektrode gekoppelt ist;
einen ersten Stromsensor, der dazu ausgelegt ist, einen ersten Strompegel zu erfassen, der mit der Ablationsenergie assoziiert ist, die von dem Generator durch die erste Körperoberflächenelektrode (144r) bereitgestellt wird;
einen zweiten Stromsensor, der dazu ausgelegt ist, einen zweiten Strompegel zu erfassen, der mit der Ablationsenergie assoziiert ist, die von dem Generator durch die zweite Körperoberflächenelektrode bereitgestellt wird; und
wobei der Stromkreis mit dem ersten Stromsensor und dem zweiten Stromsensor gekoppelt ist, wobei der Stromkreis (149) dem variablen Impedanzstromkreis ein Steuersignal bereitstellt, um den ersten Strompegel durch die erste Körperoberflächenelektrode und den zweiten Strompegel durch die zweite Körperoberflächenelektrode ins Gleichgewicht zu bringen.

9. System nach Anspruch 1, wobei der Stromkreis (149) eine einstellbare Frequenzstromquelle (310) zur Bereitstellung des Abfragesignals umfasst.

10. System nach Anspruch 1, wobei der Stromkreis (149) ein Signal mit geringer Kontaktqualität bereitstellt, wenn das Erfassungssignal höher als ein Schwellenwert ist.

11. System nach Anspruch 10, wobei der Stromkreis dazu ausgelegt ist, den Schwellenwert als Reaktion auf einen Strompegel, der mit der Ablationsenergie assoziiert ist, die von dem Generator durch die erste Körperoberflächenelektrode (144r) bereitgestellt wird, dynamisch einzustellen.

12. System nach Anspruch 11, wobei der Schwellenwert niedriger eingestellt wird, wenn der Strompegel, der mit der Ablationsenergie assoziiert ist, die von dem Generator (122) durch die erste Körperoberflächenelektrode (144r) bereitgestellt wird, erhöht ist, oder
wobei der Stromkreis (149) einen Stromwandler umfasst, der zwischen einem Rückleitungsknoten des Generators (122) und der ersten Körperoberflächenelektrode (144r) angeordnet ist, wobei der Strompegel unter Verwendung eines Stromsignals von dem Stromwandler bestimmt wird, oder
wobei der Stromkreis einen Analog-Digital-Wandler zum Empfangen des Erfassungssignals umfasst.

13. System nach Anspruch 1, wobei der Stromkreis einen einstellbaren Impedanzstromkreis in Reihe mit der ersten Körperoberflächenelektrode und einen Rückleitungsknoten des Generators umfasst, wobei der einstellbare Impedanzstromkreis dazu abgestimmt ist, Signale in einem ersten Frequenzbereich zu blockieren, während er Signale in einem zweiten Frequenzbereich durchlässt.

14. System nach Anspruch 1, das ferner Folgendes umfasst:
einen Katheter, der dazu ausgelegt ist, einem Körper die Ablationsenergie bereitzustellen; wobei die erste Körperoberflächenelektrode (144r) eine Rückleitungselektrode ist.

## Revendications

1. Système de surveillance de la qualité de contact d'une première électrode destinée à se trouver en contact avec la surface d'un corps, le système comprenant :
une première électrode destinée à se trouver en contact avec la surface d'un corps (144r) ;
un générateur (122) configuré pour fournir de l'énergie d'ablation ;
une unité de commande électronique (UCE) (142) configurée pour être en communication électrique avec la première électrode destinée à se trouver en contact avec la surface d'un corps (144r) et le générateur (122) ;
un circuit (149) connectant la première électrode destinée à se trouver en contact avec la surface d'un corps (144r) à l'UCE (142) ;
dans lequel l'UCE (142) est configurée pour envoyer un signal d'interrogation à travers le circuit (149) à la première électrode destinée à se trouver en contact avec la surface d'un corps (144r) et recevoir un signal de détection provenant de la première électrode destinée à se trouver en contact avec la surface d'un corps (144r) à travers le circuit (149) en réponse au signal d'interrogation ; et
dans lequel l'UCE (142) détermine la qualité de contact de la première électrode destinée à se trouver en contact avec la surface d'un corps (144r) en traitant le signal de détection, **caractérisé en ce que** le signal d'interrogation est fourni à une première fréquence si l'énergie d'ablation est fournie et à une deuxième fréquence si l'énergie d'ablation n'est pas fournie.

2. Système selon la revendication 1, dans lequel la première fréquence est inférieure à la deuxième fréquence, ou dans lequel la première fréquence est comprise entre 10 kHz et 20 kHz et la deuxième fréquence est comprise entre 20 kHz et 100 kHz.

3. Système selon la revendication 1, dans lequel la première électrode destinée à se trouver en contact avec la surface d'un corps (144r) comprend une première partie conductrice et une deuxième partie conductrice, dans lequel la première partie conductrice est séparée de la deuxième partie conductrice par un espace, dans lequel le signal d'interrogation est envoyé à travers l'espace et le signal de détection a une tension correspondant à une impédance à travers l'espace.

4. Système selon la revendication 3, dans lequel le circuit (149) produit un signal de faible qualité de contact lorsque le signal de détection est supérieur à un seuil, le système comprenant un circuit de déclenchement (232) configuré pour arrêter, désactiver ou empêcher la fourniture de l'énergie d'ablation par le générateur en réponse au signal de faible qualité de contact.

5. Système selon la revendication 1, comprenant en outre une deuxième électrode destinée à se trouver en contact avec la surface d'un corps, dans lequel la première électrode destinée à se trouver en contact avec la surface d'un corps et la deuxième électrode destinée à se trouver en contact avec la surface d'un corps comprennent chacune une première partie conductrice et une deuxième partie conductrice, chaque première partie conductrice étant séparée de chaque deuxième partie conductrice par un espace respectif, dans lequel le circuit (149) fournit des signaux d'interrogation respectifs à, et reçoit des signaux de détection respectifs provenant de, chaque première partie conductrice et chaque deuxième partie conductrice.

6. Système selon la revendication 5, dans lequel le circuit (149) fournit chacun des signaux d'interrogation à une première fréquence si l'énergie d'ablation est fournie par le générateur et à une deuxième fréquence si l'énergie d'ablation n'est pas fournie, la première fréquence étant inférieure à la deuxième fréquence, dans lequel le circuit (149) fournit un signal de faible qualité de contact en réponse au fait qu'au moins l'un des signaux de détection respectifs est supérieur à un seuil.

7. Système selon la revendication 6, dans lequel le signal de faible qualité de contact indique une réduction de plus de 40 pour cent d'une surface d'adhésion par rapport à une surface d'adhésion prévue de la première électrode destinée à se trouver en contact avec la surface d'un corps (144r) ou de la deuxième électrode destinée à se trouver en contact avec la surface d'un corps.

8. Système selon la revendication 1, comprenant en outre :
une deuxième électrode destinée à se trouver en contact avec la surface d'un corps ;
un circuit à impédance variable couplé en série avec au moins l'une de la première électrode destinée à se trouver en contact avec la surface d'un corps (144r) et de la deuxième électrode destinée à se trouver en contact avec la surface d'un corps ;
un premier capteur de courant configuré pour détecter un premier niveau de courant associé à l'énergie d'ablation fournie par le générateur à travers la première électrode destinée à se trouver en contact avec la surface d'un corps (144r) ;
un deuxième capteur de courant configuré pour détecter un deuxième niveau de courant associé à l'énergie d'ablation fournie par le générateur à travers la deuxième électrode destinée à se trouver en contact avec la surface d'un corps ; et
dans lequel le circuit est couplé au premier capteur de courant et au deuxième capteur de courant, dans lequel le circuit (149) fournit un signal de commande au circuit à impédance variable pour équilibrer le premier niveau de courant à travers la première électrode destinée à se trouver en contact avec la surface d'un corps et le deuxième niveau de courant à travers la deuxième électrode destinée à se trouver en contact avec la surface d'un corps.

9. Système selon la revendication 1, dans lequel le circuit (149) comprend une source de courant à fréquence ajustable (310) pour fournir le signal d'interrogation.

10. Système selon la revendication 1, dans lequel le circuit (149) fournit un signal de faible qualité de contact lorsque le signal de détection est supérieur à un seuil.

11. Système selon la revendication 10, dans lequel le circuit est configuré pour ajuster dynamiquement le seuil en réponse à un niveau de courant associé à l'énergie d'ablation fournie par le générateur à travers la première électrode destinée à se trouver en contact avec la surface d'un corps (144r).

12. Système selon la revendication 11, dans lequel le seuil est ajusté à un niveau inférieur lorsque le niveau de courant associé à l'énergie d'ablation fournie par le générateur (122) à travers la première électrode destinée à se trouver en contact avec la surface d'un corps (144r) est augmenté, ou
dans lequel le circuit (149) comprend un transformateur de courant disposé entre un nœud de retour du générateur (122) et la première électrode destinée à se trouver en contact avec la surface d'un corps (144r), dans lequel le niveau de courant est déterminé à l'aide d'un signal de courant provenant du transformateur de courant, ou
dans lequel le circuit comprend un convertisseur analogique-numérique pour recevoir le signal de détection.

13. Système selon la revendication 1, dans lequel le circuit comprend un circuit à impédance ajustable en série avec la première électrode destinée à se trouver en contact avec la surface d'un corps et un nœud de retour du générateur, dans lequel le circuit à impédance ajustable est accordé pour bloquer les signaux dans une première plage de fréquences tout en laissant passer les signaux dans une deuxième plage de fréquences.

14. Système selon la revendication 1, comprenant en outre :
un cathéter configuré pour appliquer l'énergie d'ablation à un corps ; dans lequel la première électrode destinée à se trouver en contact avec la surface d'un corps (144r) est une électrode de retour.
